(19) Europäisches Patentamt

European Patent Office

Office européen des brevets

(11) Veröffentlichungsnummer: **0 031 423**
**B1**

(12) # EUROPÄISCHE PATENTSCHRIFT

(45) Veröffentlichungstag der Patentschrift:
20.06.84

(51) Int. Cl.³: **C 07 C 85/24,** C 07 C 87/50,
C 07 C 85/08, C 07 C 85/26

(21) Anmeldenummer: **80106808.1**

(22) Anmeldetag: **05.11.80**

(54) Verfahren zur Herstellung von Polyphenyl-polymethylen-polyaminen.

(30) Priorität: **26.11.79 DE 2947531**

(43) Veröffentlichungstag der Anmeldung:
**08.07.81 Patentblatt 81/27**

(45) Bekanntmachung des Hinweises auf die Patenterteilung:
**20.06.84 Patentblatt 84/25**

(84) Benannte Vertragsstaaten:
**BE DE FR GB IT NL**

(56) Entgegenhaltungen:
**EP - A - 0 003 303
GB - A - 1 228 495
US - A - 2 938 054**

(73) Patentinhaber: **BASF Aktiengesellschaft,
Carl-Bosch-Strasse 38, D-6700 Ludwigshafen (DE)**

(72) Erfinder: **Koehler, Waldemar, Dr.,
Max-Slevogt-Strasse 28, D-6710 Frankenthal 1 (DE)**
Erfinder: **Langensiepen, Hans-Werner, Dr.,
Limburgstrasse 87, D-6702 Bad Duerkheim 1 (DE)**
Erfinder: **Mueller, Berthold, Dr., An der Bleiche 12,
D-6701 Erpolzheim (DE)**

ACTORUM AG

## Beschreibung

Die Erfindung betrifft ein Verfahren zur Herstellung von Polyphenyl-polymethylen-polyaminen mit einem Gehalt an Diamino-diphenylmethan-Isomeren von 55 bis 85 Gew.-%, bezogen auf das Gesamtgewicht, und einem Anteil an 4,4'-Diamino-diphenylmethan von ungefähr 95 bis 99 Gew.-%, bezogen auf das Gesamtgewicht an Diamino-diphenylmethan-Isomeren, durch mehrstufige Kondensation von Anilin mit Formaldehyd in Gegenwart einer bei 25°C zu mindestens 80% gesättigten, wässrigen Aminhydrochloridlösung, die mindestens 85 Gew.-% Anilinhydrochlorid enthält, als Katalysator, wobei die molaren Verhältnisse von Anilin : Formaldehyd 2,5 bis 3 : 1 und Chlorwasserstoff : Anilin 0,4 bis 0,9 : 1 betragen, das erhaltene Kondensationsgemisch mit 0,5 bis 1,0 Teilen Anilin pro Teil Kondensationsgemisch versetzt, diese Mischung einer Extraktion mit 0,8 bis 1,5 Teilen Wasser pro Teil Anilin/Kondensationsgemisch-Mischung unterworfen, die wässrige Extrationsphase auf einen Aminhydrochloridgehalt von mindestens 80% der Sättigungsgrenze bei 25°C eingeengt und an den Prozessanfang zurückgeführt wird und aus der organischen Phase die Polyphenyl-polymethylen-polyamine isoliert werden.

Die Herstellung von Polyphenyl-polymethylen-polyaminen, in folgendem kurz Roh-MDA genannt, durch Kondensation von Anilin mit Formaldehyd in Gegenwart von sauren Katalysatoren ist in zahlreichen Patentschriften beschrieben worden und liefert je nach Verfahrensweise, d.h. nach gewählten Mengenverhältnissen der Ausgangskomponenten und Reaktionsparametern, unterschiedlich zusammengesetzte Produkte. So erhält man bei der Kondensation in Gegenwart von schwach sauren Katalysatoren Roh-MDA-Gemische mit einem relativ hohen Anteil an 2,2'- und 2,4'-Diamino-diphenylmethanen, während Roh-MDA-Gemische mit einem grossen Gehalt an 4,4'-Diamino-diphenylmethan und gleichzeitig geringem Anteil an 2,4'-Diamino-diphenylmethan nur in Gegenwart von grösseren Mengen stark saurer Katalysatoren, vorzugsweise von starken Mineralsäuren, wie Salzsäure, hergestellt werden können.

Der Gehalt an Diamino-diphenylmethan-Isomeren in Roh-MDA-Gemischen ist ferner abhängig vom Anilin-Formaldehyd-Verhältnis und der Kondensationstemperatur, wobei grosse Anilin-Formaldehyd-Verhältnisse und niedrige Kondensationstemperaturen hohe Diamino-diphenylmethananteile ergeben (BE-PS 648 787, CA-PS 700 026).

Der Vorteil der hohen Selektivität der Salzsäure für die Bildung von 4,4'-Diamino-diphenylmethan muss mit dem Verlust des Katalysators erkauft werden, da dieser üblicherweise nach bekannten Verfahren mit Basen neutralisiert und aus dem Reaktionsgemisch abgetrennt wird. Nachteilig ist ferner, dass die anfallenden Salzlösungen technisch nicht sinnvoll verwertet werden können und die Umwelt belasten.

Nach Angaben der GB-A-1 228 495 werden in Lösungsmittel gut lösliche Methylenbrücken aufweisende Polyarylamine unter Verwendung eines niedrigen Amin-Formaldehyd-Verhältnisses hergestellt, indem man die Aufbaukomponenten Arylamin, Formaldehyd und Katalysator in einen Mischkessel einbringt und diese Mischung mit einem kontinuierlichen Strom aus teilweise aber unvollständig reagiertem Formaldehyd, Arylamin und Katalysator rückvermischt. Eine Katalysatorextraktion und -rückführung wird nicht beschrieben. Nachteilig ist auch bei diesem Verfahren, dass der saure Katalysator nach beendeter Kondensation mit Basen neutralisiert und die gebildeten Salze entsorgt werden müssen. Das Verfahren ist daher umweltfeindlich und wenig wirtschaftlich.

Zur Beseitgung dieses Nachteils wird nach Angaben der DE-AS-2 227 110 (US 4 025 557) die Kondensation in Gegenwart von soviel Wasser durchgeführt, dass ein inhomogenes Reaktionsgemisch erhalten wird, das sich in zwei Phasen scheidet. Die organische Phase wird ohne Alkalibehandlung mit Wasser gewaschen und der destillativen Aufarbeitung zugeführt, während die wässrige Phase nach Zugabe von als Ausgangsmaterial verwendetem Amin und Formaldehyd an den Prozessanfang zurückgeführt wird. Gemäss DE-AS-2 238 920 (US 3 996 283) wird das ausreagierte wässrige Kondensationsgemisch mit einem hydrophoben Lösungsmittel extrahiert, die so erhaltene Lösungsmittelphase zu Roh-MDA aufgearbeitet und die den sauren Katalysator enthaltende wässrige Phase nach Zugabe von aromatischem Amin und Formaldehyd an den Prozessanfang zurückgeführt. Eine weitere Verfahrensvariante wird in der DE-AS-2 426 116 (GB-PS 1 450 632) beschrieben. Nach diesem Verfahren werden zunächst das aromatische Amin und Formaldehyd in Abwesenheit eines Katalysators unter Bildung von Vorkondensaten und Abtrennung des vorhandenen Wassers kondensiert, danach das erhaltene Vorkondensat in Gegenwart von 0,001 bis 0,5 Mol wässriger saurer Salze der aromatischen Amine, gegebenenfalls in Gegenwart von polaren organischen Lösungsmitteln, umgesetzt und der eingesetzte Katalysator mit Wasser ausgewaschen und wiederverwendet.

Die genannten Verfahren weisen den Nachteil auf, dass zur Extraktion vorwiegend organische hydrophobe Lösungsmittel oder gegebenenfalls Mischungen aus Wasser und hydrophoben Lösungsmitteln verwendet werden. Der Lösungsmittelzusatz ist jedoch nicht nur kostspielig, sodern erfordert auch zusätzliche Verfahrensmassnahmen, z.B. einen Lösungsmittelkreislauf mit Destillationsstufen. Apparativ aufwendig ist es, die Kondensation in heterogener Phase durchzuführen, da hierzu grosse Wassermengen erhitzt und gefördert werden müssen. Nach den beschriebenen Verfahren werden ferner Roh-MDA-Gemische mit relativ hohen Gehalten an 2,4'--Diamino-diphenylmethan erhalten.

Die Aufgabe der vorliegenden Erfindung bestand darin, ein umweltfreundliches wirtschaftliches Verfahren zur Herstellung von Roh-MDA-Gemischen zu entwickeln, die einen grossen Gehalt an Diamino-diphenylmethan-Isomeren und hohen 4,4'-Diamino--diphenylmethananteil besitzen.

Diese Aufgabe konnte überraschenderweise gelöst werden durch ein Verfahren zur Herstellung von Roh-MDA mit einem grossen Gehalt an Diamino-diphenylmethan-Isomeren und einem hohen 4,4'-Diamino-diphenylmethananteil durch mehrstufige Kondensation von Anilin mit Formaldehyd in Gegenwart von Aminhydrochlorid als Katalysator, das dadurch gekennzeichnet ist, dass man

a) die Einsatzstoffe Anilin, Formaldehyd und Chlorwasserstoff in solchen Mengen verwendet, dass die molaren Verhältnisse von Anilin : Formaldehyd 2,5 bis 3 : 1 und von Chlorwasserstoff : Anilin 0,4 bis 0,9 : 1 betragen,

b) als Katalysator eine bei 25°C zu mindestens 80% gesättigte, wässrige Aminhydrochloridlösung verwendet, die bezogen auf den Aminhydrochloridgehalt 85 bis 100 Gew.-% Anilinhydrochlorid enthält,

c) Anilin, Formaldehyd und die wässrige Aminhydrochloridlösung kontinuierlich der Reaktionsmischung der ersten Kondensationsstufe bei einer Reaktionstemperatur von maximal 50°C zumischt und die Kondensation in mehreren Stufen zu Ende führt,

d) das erhaltene Kondensationsgemisch mit 0,5 bis 1,0 Teilen Anilin pro Teil Kondensationsgemisch versetzt und diese Mischung einer Extraktion mit Wasser unterwirft, wobei 0,8 bis 1,5 Teile Wasser auf ein Teil Anilin/Kondensationsgemisch-Mischung verwendet werden,

e) die wässrige Extraktionsphase auf einen Aminhydrochloridgehalt von mindestens 80% der Sättigungsgrenze bei 25°C einengt und an den Prozessanfang zurückführt und

f) aus der organischen Phase die Polyphenyl-polymethylen-polyamine isoliert.

Mit Hilfe des erfindungsgemässen Verfahrens können Roh-MDA-Gemische hergestellt werden, die einen Gehalt an Diamino-diphenylmethan-Isomeren von 55 bis 85 Gew.-%, vorzugsweise 60 bis 80 Gew.-%, bezogen auf das Gesamtgewicht, und einen 4,4'-Diamino-diphenylmethananteil von 95 bis 99 Gew.-%, vorzugsweise 97 bis 99 Gew.-%, bezogen auf das Gesamtgewicht an Diamino-diphenylmethan-Isomeren, besitzen.

Das erfindungsgemässe Verfahren weist den Vorteil auf, dass die Kondensation von Anilin mit Formaldehyd in Gegenwart von Aminhydrochlorid in homogener Phase verläuft und der Katalysator, obgleich er in hohen Konzentrationen vorliegt, nach Zugabe von Anilin mit Wasser extrahiert werden kann. Hydrophobe organische Lösungsmittel sind hierzu nicht erforderlich. Als Katalysator zurückgeführt wird ein Aminhydrochlorid, das zu 85 bis 100 Gew.-% aus als Einsatzstoff verwendbarem Anilinhydrochlorid besteht und nur in untergeordneten Mengen Hydrochloride von bereits gebildetem Roh-MDA enthält. Die Raum-Zeit-Ausbeute wird dadurch beträchtlich erhöht. Überraschend war jedoch insbesondere, dass durch die Verwendung von Aminhydrochlorid, das neben Roh-MDA-Hydrochloriden mindestens 85 Gew.-% Anilinhydrochlorid, bezogen auf das Gesamtgewicht, enthält, als Katalysator die Bildung von Diamino-diphenylmethan-Isomeren mit hohem 4,4'-Diamino-diphenylmethananteil positiv beeinflusst werden kann. Bei separater Zugabe von Anilin und Chlorwasserstoff werden keine derart vorteilhafte Ergebnisse erzielt.

Zu den Einsatzstoffen für das erfindungsgemässe Verfahren möchten wir folgendes ausführen:

Anilin wird in der handelsüblichen Qualität verwendet. Der Formaldehyd wird vorzugsweise in Form einer wässrigen Lösung, beispielsweise als handelsübliche 30 bis 37%ige wässrige Lösung, eingesetzt. Auch an die Art und Konzentration des Chlorwasserstoffs werden keine besonderen Anforderungen gestellt. Der Chlorwasserstoff kann sowohl gasförmig als auch in Form einer wässrigen Lösung verwendet werden. Vorzugsweise zur Anwendung kommt wässrige Salzsäure in Konzentrationen von etwa 25 bis 31 Gew.-%.

Wie bereits ausgeführt wurde, wird als Katalysator eine wässrige Aminhydrochloridlösung verwendet, die bei 25°C zu mindestens 80%, vorzugsweise zu mehr als 90% und insbesondere vollständig gesättigt ist. Die gesättigte wässrige Lösung besitzt einen Aminhydrochloridgehalt von ungefähr 50 Gew.-%, bezogen auf das Gesamtgewicht. Das Aminhydrochlorid enthält, bezogen auf das Gesamtgewicht, 85 bis 100 Gew.-%, vorzugsweise 85 bis 95 Gew.-% Anilinhydrochlorid. Reines Anilinhydrochlorid kommt zur Anwendung, wenn der Katalysator in einer unabhängigen Verfahrensstufe vor der Kondensationsreaktion aus Anilin und Chlorwasserstoff hergestellt wird.

Vorzugsweise als Katalysator verwendet wird jedoch Aminhydrochlorid, das aus der Extraktionsstufe als wässrige Lösung zurückgeführt wird und im wesentlichen besteht aus 85 bis 100 Gew.-%, vorzugsweise 85 bis 95 Gew.-% Anilinhydrochlorid und 15 bis 0 Gew.-%, vorzugsweise 5 bis 15 Gew.-% Hydrochloriden von Roh-MDA, insbesondere von Diamino-diphenylmethanen.

Die Einsatzstoffe Anilin, Formaldehyd und Chlorwasserstoff kommen in solchen Mengen zur Anwendung, dass die molaren Verhältnisse von Anilin : Formaldehyd 2,5 bis 3 : 1 und von Chlorwasserstoff : Anilin 0,4 bis 0,9 : 1, vorzugsweise 0,6 bis 0,9 : 1, betragen. Unter dem Verhältnis Anilin : Formaldehyd ist im eigentlichen Sinne der Erfindung das molare Verhältnis von Anilin plus Anilinhydrochlorid zu Formaldehyd zu verstehen.

Mit nachstehender Figur, die ein Blockschema zeigt, soll das erfindungsgemässe Verfahren beispielhaft erläutert werden.

Gemessene und geregelte Ströme von Anilin über Zuleitung (1), wässriger Formaldehydlösung über Zuleitung (2) und wässrige Aminhydrochloridlösung über Zuleitung (12) werden kontinuierlich der ersten Kondensationsstufe der mehrstufigen Kondensation (K) zugeführt. Nach beendeter Kondensation gelangt das erhaltene Kondensationsgemisch über die Ab- bzw. Zuleitung (3) in den Mischer (M) und wird dort mit Anilin, das über die zuleitung (5) hinzugefügt und wässrigem Anilin, das über die Zuleitung (4) rückgeführt wird, gemischt. Die Anilin-Kondensationsgemisch-Mischung wird über Ab- bzw. Zuleitung (6) dem Extraktor (E) zugeführt und mit Wasser, das über Zuleitung (14) hinzugefügt wird, extrahiert. Die wässrige Phase, die nahezu das gesamte Aminhydrochlorid enthält, gelangt über Ab- bzw. Zuleitung (8) in den Verdampfer (V), wird dort aufkonzentriert, wobei das abgetrennte anilinhaltige Wasser in den Abscheider (S) abgeführt und das Konzentrat über die Zuleitung (12) in die erste Kondensationsstufe der mehrstufigen Kondensation zurückgeführt wird. Die organische Phase der Extraktion (E), die im wesentlichen aus Roh-MDA, Anilin, Wasser und gegebenenfalls Spuren Aminhydrochlorid besteht, gelangt über die Ab- bzw. Zuleitung (7) in die Destillation (D), in der das Roh-MDA als Sumpfprodukt abgetrennt und über die Ableitung (13) abgeführt wird. Das abdestillierte Anilin und Wasser zerfällt nach der Kondensation in zwei Flüssigphasen, von denen die Anilin-Phase über die Leitung (4) in den Mischer (M) zurückgeführt und die Wasser-Phase über die Leitung (9) abgezogen wird.

Sofern als Katalysator reines Anilinhydrochlorid verwendet wird, insbesondere beim Anfahren der Kondensationsreaktion, wird dieses im Vormischer (VM) aus Anilin und Chlorwasserstoff, die über die Zuleitungen (15) und (16) geführt werden, hergestellt. Das Anilinhydrochlorid gelangt über die Ab- bzw. Zuleitungen (17) und (12) in die erste Kondensationsstufe der mehrstufigen Kondensation (K). Über die Zuleitung (16), den Vormischer (VM) und die Ab- bzw. Zuleitung (17) kann der Aminhydrochloridlösung im Verlaufe der Kondensation gegebenenfalls zusätzlich Chlorwasserstoff zugegeben werden.

Zu den einzelnen Verfahrensstufen und den erforderlichen Reaktionsbedingungen des erfindungsgemässen Verfahrens möchten wir ergänzend noch das folgende ausführen:

Wie bereits dargelegt wurde, wird die Kondensation von Anilin und Formaldehyd in Gegenwart von Aminhydrochlorid als Katalysator in mehreren Stufen, beispielsweise 2 bis 5, vorzugsweise 3 bis 4 und insbesondere 4 Stufen durchgeführt. Die einzelnen Kondensationsstufen entsprechen im allgemeinen räumlich den einzelnen Rührkesseln einer Rührkesselkaskade, die beim erfindungsgemässen Verfahren vorzugsweise aus 4 hintereinandergeschalteten Rührkesseln besteht. Die einzelnen hintereinandergeschalteten Rührkessel können in ihren Dimensionen und Füllmengen vorteilhafterweise so gewählt werden, dass in jedem Kessel die mittlere Verweilzeit gleich oder annähernd gleich ist und die Gesamtverweilzeit zweckmässigerweise 2,0 bis 6,0 Stunden, vorzugsweise 2,0 bis 4,0 Stunden beträgt.

Als Reaktionsgefässe müssen jedoch nicht notwendigerweise Rührkessel verwendet werden. Nach der 1. Reaktionsstufe werden immer homogene Flüssigphasen erhalten; für diese Stufen sind daher alle Reaktionsapparate geeignet, die mit Einrichtungen zur Zu- und Abfuhr von Wärme ausgerüstet sind und in denen die Durchmischung für die Aufrechterhaltung einer gleichmässigen Temperaturverteilung ausreicht. So können z.B. auch Reaktionstürme oder Rohrreaktoren zum Einsatz kommen. Lediglich im Hinblick auf das 1. Reaktionsgefäss ist darauf zu achten, dass die Ausgangsströme (1), (2) und (12) innig miteinander vermischt werden und dass bei Anwendung bestimmter molarer Einsatzzahlen von Anilin : Formaldehyd und Salzsäure : Anilin innerhalb des beanspruchten Bereichs das Reaktionsgemisch der 1. Reaktionsstufe als Feststoffsuspension vorliegen kann. In diesem Fall ist eine wirksame mechanische Rührung erforderlich, so dass sich ein Rührkessel oder ein Mischkreis mit Zwangsumlauf als Reaktionsgefäss anbietet. Die Durchmischung der Ausgangsströme (1), (2) und (12) kann auch vor dem Eintritt in das 1. Reaktionsgefäss erfolgen. Falls ausserdem das Reaktionsgemisch in der 1. Reaktionsstufe eine homogene Flüssigphase bildet, gilt für diese Stufe das gleiche wie für die nachfolgenden bereits ausgeführt.

Von Bedeutung ist neben der Einhaltung der Verweilzeit ferner die Reaktionstemperatur, die von der ersten zur letzten Kondensationsstufe stufenweise erhöht wird und bei einer 4stufigen Kondensation in der ersten Kondensationsstufe (bzw. im ersten Rührkessel) 20° bis 50°C, vorzugsweise 30 bis 40°C, in der zweiten Kondensationsstufe (bzw. im zweiten Rührkessel) 40° bis 70°C, vorzugsweise 50 bis 60°C, in der dritten Kondensationsstufe (bzw. im dritten Rührkessel) 60° bis 90°C, vorzugsweise 70 bis 80°C und in der vierten Kondensationsstufe (bzw. im vierten und gegebenenfalls weiteren Rührkesseln) 90° bis 110°C beträgt. Hierbei ist insbesondere zu beachten, dass die maximale Reaktionstemperatur in der ersten Kondensationsstufe von 50°C nicht überschritten wird.

Von untergeordneter Bedeutung für die Zusammensetzung des erfindungsgemäss hergestellten Roh-MDA-Gemisches ist hingegen der Wassergehalt der Kondensationsmischung, wobei jedoch unnötig grosse Wassermengen die Raum-Zeitausbeute verringern und die Verfahrens-, insbesondere die Energiekosten, erhöhen. Es hat sich daher als vorteilhaft erwiesen, den Wassergehalt so einzustellen, dass die Kondensation in homogener Phase erfolgt, die erhaltenen Kondensationsgemische nach beendeter Kondensation, d.h. nach quantitativer Umsetzung des Formaldehyds und mindestens 98%iger Umlagerung der Zwischenprodukte vom Aniltyp in methylenbrückenhaltige Roh-MDA-Gemische, Wassergehalte von 35 bis 60 Gew.-%, vorzugsweise 40 bis 55 Gew.-%, und Chlorwasserstoffgehalte von 6 bis 13 Gew.-%, vorzugsweise von 8 bis 12 Gew.-%, aufweisen, wobei die Gewichtsprozente bezogen sind auf das Gesamtgewicht des Kondensationsgemisches.

Ein Gewichtsteil des erhaltenen Kondensationsgemisches wird mit 0,5 bis 1,0 Gew.-Teilen, vorzugsweise 0,8 bis 1,0 Gew.-Teilen Anilin im Mischer (M) gemischt. Hierzu verwendet wird frisches Anilin aus der Zuleitung (5) und aus der Destillation (D) über Ab- bzw. Zuleitung (4) zurückgeführtes Anilin.

Die hydrochloridhaltige Anilin-Kondensationsgemisch-Mischung, die eine Chloridkonzentration von ungefähr 3 bis 9 Gew.-%, vorzugsweise von 5 bis 9 Gew.-%, besitzt, wird zur Abtrennung der Aminhydrochloride einer mehrstufigen Extraktion, vorzugsweise einer mehrstufigen Gegenstromextraktion, mit Wasser unterworfen. Bei Verwendung von Extraktoren mit 4 bis 9, vorzugsweise 5 bis 7 theoretischen Trennstufen wird eine effektive Trennung erzielt, wenn pro Gew.-Teil Anilin-Kondensationsgemisch-Mischung mit 0,8 bis 1,5 Gew.-Teilen, vorzugsweise mit 1,0 bis 1,2 Gew.-Teilen Wasser, das über Zuleitung (14) zufliesst, extrahiert wird. Geeignete Extraktionstemperaturen können beispielsweise zwischen 15° und 80°C liegen, wobei Temperaturen von 20 bis 40°C bevorzugt Anwendung finden.

Die wässrige Phase der Extraktion, die eine Aminhydrochloridkonzentration (gerechnet als Anilinhydrochlorid) von 90 bis 16,5 Gew.-%, vorzugsweise von 11,0 bis 14,0 Gew.-%, aufweist, und wobei die Aminhydrochloride im wesentlichen bestehen aus 85 bis 95 Gew.-%, vorzugsweise 85 bis 90 Gew.-% Anilinhydrochlorid und 15 bis 5 Gew.-%, vorzugsweise 15 bis 10 Gew.-% Hydrochloriden des Roh-MDA's, wird im Verdampfer (V) aufkonzentriert. Hierbei verdampfen Wasser und Anilin, die nach der Konzentration im Abscheider (S) in zwei Flüssigphasen zerfallen, von denen die Anilin-Phase über die Leitung (11) und die Wasser-Phase über die Leitung (10) abgezogen werden. Die Anilin-Phase kann selbstverständlich dem Mischer (M) zugeführt werden. Vorzugsweise werden die wässrigen Aminhydrochloridlösungen bis zur Sättigungsgrenze bei 25°C eingeengt und weisen dann einen Aminhydrochloridgehalt von ungefähr 50 Gew.-% auf. Gegebenenfalls kann es jedoch auch zweckmässig sein, nicht bis zur Sättigungsgrenze, sondern nur bis zu mindestens 80%, vorzugsweise zu mehr als 90% der Sättigungsgrenze bei 25°C einzuengen. Dies kann beispielsweise vorteilhaft sein, wenn als Einsatzstoff wässrige Formaldehydlösungen mit Formaldehydgehalten bis zu 50 Gew.-% Verwendung finden. Die aufkonzentrierten Aminhydrochloridlösungen werden als Katalysatoren an den Prozessanfang zurückgeführt.

Aus der organischen Phase der Extraktion (E), die eine durchschnittliche Chlorkonzentration kleiner als 0,005 Gew.-%, vorzugsweise kleiner als 0,003 Gew.-% besitzt, und im wesentlichen besteht aus 20 bis 25 Gew.-% Roh-MDA, 65 bis 80 Gew.-% Anilin und 3 bis 10 Gew.-% Wasser wird das Roh-MDA nach bekannten Methoden, beispielsweise durch fraktionierte Destillation unter vermindertem Druck oder Dünnschichtdestillation vom Anilin und Wasser abgetrennt. Das Roh-MDA wird über die Ableitung (13) dem Vorratsbehälter zugeführt. Das wasserhaltige Anilin zerfällt nach der Kondensation im Abscheider (S') in eine Anilinphase, die über Zuleitung (4) in den Mischer (M) zurückgeführt wird, während die Wasserphase über die Leitung (9) abgezogen wird. Die beiden Abwasserströme (9) und (10) sind mit Anilin gesättigt. Dieses anilinhaltige Wasser kann zur Deckung des Wasserstroms (14) verwendet werden. Da jedoch mit der Formaldehydlösung (2) Wasser in den Prozess eingetragen wird und während der Reaktion Wasser entsteht, muss zur Aufrechterhaltung der Mengenbilanz ein Teil des anfallenden Abwassers aus dem Prozess ausgeschleust werden (z.B. über Ableitung 10).

Die erfindungsgemäss hergestellten Polyphenyl--polymethylen-polyamine werden vorzugsweise durch Umsetzung mit Phosgen in die entsprechenden Polyphenyl-polymethylen-polyisocyanat-Mischungen übergeführt, aus denen die Diisocyanato--diphenylmethan-Isomeren und insbesondere das 4,4'-Diisocyanato-diphenylmethan durch Destillation und/oder Kristallisation abgetrennt werden. Es ist jedoch auch möglich, die Polyphenyl-polymethylen-polyamine selbst durch Destillation oder Kristallisation zu trennen. Polyphenyl-polymethylen-polyamine und insbesondere die Diamino-diphenylmethan-Isomere sind wertvolle Zwischenprodukte für Farbstoffe und Kunststoffe. 4,4'-Diamino-dipnenylmethan eignet sich beispielsweise als Härter für Epoxidharze und als Vernetzungsmittel in der Polyurethanchemie.

*Beispiel 1*

Die Apparatur besteht aus einer Laborrührkesselkaskade aus 4 hintereinandergeschalteten Rührkolben mit einem Rauminhalt von jeweils 1,0 Litern. Als Mischer wird ein Rührkolben mit einem Rauminhalt von 1,0 Litern verwendet. Die kontinuierliche Gegenstromextraktion wird in 2 Labordrehscheibenkolonnen, die einen Durchmesser von 42 mm haben und je 18 Rührzellen besitzen, bei 300 Umdrehungen pro Minute durchgeführt. Die wässrige Extraktionsphase wird in einem Rotationsverdampfer eingeengt. Die organische Extraktionsphase wird in einer Destillationskolonne zweistufig unter vermindertem Druck fraktioniert.

Im Zuge des kontinuierlichen Betriebs werden in den 1. Rührkessel, der der ersten Kondensationsstufe entspricht, stündlich über die Zuleitung (1) 112 g (1,21 Mol) Anilin, die Zuleitung (2) 122 g (1,22 Mol) 30 gew.-%iger wässriger Formaldehyd und die Zuleitung (12) 542 g einer wässrigen Aminhydrochloridlösung, die besteht aus 172 g (1,84 Mol) Anilin, 21 g (0,1 Mol) Roh-MDA, 72 g (1,95 Mol) Chlorwasserstoff und 278 g Wasser, eingebracht. Die Reaktionstemperatur im 1. Rührkolben beträgt 40°C und die Verweilzeit ungefähr 60 Minuten.

Die Kondensation des Reaktionsgemisches wird im 2. bis 4. Rührkolben, die der 2. bis 4. Kondensationsstufe entsprechen, zu Ende geführt. Die Reaktionstemperaturen und Verweilzeiten betragen im 2. Rührkolben 50°C, ca. 60 Minuten, im 3. Rührkolben 80°C, ca. 60 Minuten und im 4. Rührkolben 103°C und ca. 60 Minuten.

Dem Mischer (M) werden über die Ab- bzw. Zuleitung (3) stündlich aus der Kondensation (K) 775 g Kondensationsgemisch mit einer Temperatur von ungefähr 100°C zugeführt. Stündlich zugegeben werden dem Kondensationsgemisch über die Zuleitung (5) 120 g (1,28 Mol) Anilin und die Zuleitung (4) 625 g wassergesättigtes Anilin. Die Temperatur im Mischer beträgt 40°C.

Der mehrstufigen Gegenstromextraktion werden über Ab- bzw. Zuleitung (6) stündlich 1520 g einer Anilin/Kondensationsgemisch-Mischung, die besteht aus 17,3 Gew.-% Roh-MDA, 51,0 Gew.-% Anilin, 4,7 Gew.-% HCl und 27 Gew.-% Wasser und über Zuleitung (4) 1520 g Wasser zugeführt. Die Extraktion erfolgt bei einer Temperatur von 25°C. Stündlich abgeführt über die Leitung (8) werden 2046 g wässrige Extraktionsphase, die besteht aus 1,0 Gew.-% Roh-MDA, 11,3 Gew.-% Anilin, 3,5 Gew.-% HCl und 84,2 Gew.-% Wasser aus der im Verdampfer (V) bei Normaldruck 1400 g Wasser und ca. 60 g Anilin stündlich abdestilliert und über Ableitung (11) abgeführt werden.

Aus dem Extraktor (E) abgeführt über die Leitung (7) werden ferner stündlich 994 g organische Extraktionsphase, die besteht aus 22 Gew.-% Roh-MDA, 62,5 Gew.-% Anilin und 14,5 Gew.-% Wasser.

Die organische Extraktionsphase wird zweistufig, zunächst bei 85°C und 72 mbar (azeotropes Abdestillieren des Anilins) und danach bei 54°C und 5,3 mbar fraktioniert destilliert, wobei als nicht destillierte Bodenfraktion stündlich 242 g (1,2 Mol) Roh-MDA (Ableitung 13), als Kopffraktion 127 g Wasser mit ca. 4 Gew.-% Anilin (Ableitung 9) und 625 g wasserhaltiges Anilin (Leitung 4) erhalten werden.

Das erhaltene Roh-MDA enthält, bezogen auf das Gesamtgewicht 78 Gew.-% Diaminodiphenylmethan-Isomere mit einem 4,4'-Diamino-diphenylmethananteil von 98 Gew.-%, bezogen auf das Gesamtgewicht an Diaminodiphenylmethan-Isomeren.

*Beispiele 2 bis 5*

Das Roh-MDA wird in der in Beispiel 1 beschriebenen Apparatur hergestellt. Die Mengen der erhaltenen Endprodukte sowie deren Zusammensetzung sind in der folgenden Tabelle zusammengefasst.

| Beispiele | | 2 | 3 | 4 | 5 |
|---|---|---|---|---|---|
| Zugabe zu 1. Rührkolben (K) | | | | | |
|    Anilin (1) | g/h | 64 | 80 | 195 | 175 |
|    30%ige Formaldehydlösung (2) | g/h | 110 | 100 | 120 | 140 |
|    Aminhydrochloridlösung (12) | g/h | 620 | 614 | 450 | 455 |
|    davon Hydrochlorid (gerechnet | | | | | |
|    als Anilinhydrochlorid) | Gew.-% | 48,4 | 48,9 | 49 | 49 |
| Reaktionstemperatur | | | | | |
|    1. Rührkolben | °C | | 40 | | |
|    2. » | » | | 50 | | |
|    3. » | » | | 80 | | |
|    4. » | » | | 100 | | |
| Gesamtverweilzeit in allen 4 Rührkolben | min. | 125 | 120 | 251 | 262 |
| HCl-Gehalt des Kondensationsgemisches (5) | Gew.-% | 10,8 | 10,7 | 8,0 | 8,1 |
| Anilin-Zugabe zum Mischer (M) | | | | | |
|    Frisch-Anilin (5) | g/h | 150 | 105 | 40 | 85 |
|    Rück-Anilin (4) | g/h | 625 | 620 | 620 | 625 |
|    wassergesättigt | | | | | |
| Zugabe zur Extraktion (E) | | | | | |
|    Ausgangsgemisch (6) | g/h | 1490 | 1520 | 1420 | 1500 |
|    Wasser (14) | g/h | 1570 | 1500 | 1500 | 1500 |
| Extraktionstemperatur | °C | | Raumtemperatur 22-25°C | | |
| Extraktphase (8) | g/h | 2150 | 2100 | 1980 | 2000 |
| Raffinatphase (7) | g/h | 910 | 920 | 940 | 1000 |
| Produktmenge (13) | g/h | 220 | 194 | 240 | 272 |
| davon Diamino-diphenylmethan | Gew.-% | 74,5 | 78,8 | 75,0 | 70,4 |
| mit einem Gehalt an 4,4'-Isomeren | Gew.-% | 96,6 | 96,2 | 92,5 | 95,5 |

*Vergleichsbeispiel*

Man verfährt analog den Angaben von Beispiel 2, verwendet jedoch als Katalysator Chlorwasserstoff, der separat dem ersten Rührkessel zugeführt wird. Im einzelnen werden in den 1. Rührkessel eingebracht: über Zuleitung (1) 260 g (2,8 Mol) Anilin, Zuleitung (2) 110 g (1,1 Mol) 30%iger wässriger Formaldehyd und Zuleitung (12) 263 g (2,22 Mol) Chlorwasserstoff (als 30,8%ige Säure).

Das erhaltene Roh-MDA besitzt einen Gehalt an Diamino-diphenylmethan-Isomeren von 72 Gew.-%, bezogen auf das Gesamtgewicht, und einen 4,4'-Diamino-diphenylmethan-Anteil von 93,6 Gew.-%, bezogen auf das Gesamtgewicht an Diamino-diphenylmethan-Isomeren.

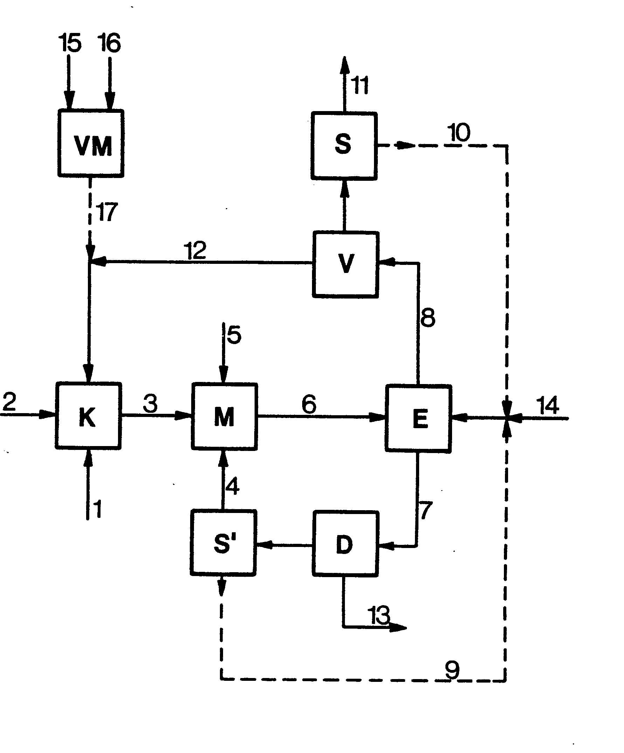

**Patentansprüche**

1. Verfahren zur Herstellung von Polyphenyl-polymethylenpolyaminen mit einem grossen Gehalt an Diaminodiphenylmethan-Isomeren und einem hohen 4,4'-Diaminodiphenylmethananteil durch mehrstufige Kondensation von Anilin und Formaldehyd in Gegenwart von Aminhydrochlorid als Katalysator, dadurch gekennzeichnet, dass man

a) die Einsatzstoffe Anilin, Formaldehyd und Chlorwasserstoff in solchen Mengen verwendet, dass die molaren Verhältnisse von Anilin : Formaldehyd 2,5 bis 3 : 1 und von Chlorwasserstoff : Anilin 0,4 bis 0,9 : 1 betragen,

b) als Katalysator eine bei 25°C zu mindestens 80% gesättigte, wässrige Aminhydrochloridlösung verwendet, die bezogen auf den Aminhydrochloridgehalt 85 bis 100 Gew.-% Anilinhydrochlorid enthält,

c) Anilin, Formaldehyd und die wässrige Aminhydrochloridlösung kontinuierlich der Reaktionsmischung der ersten Kondensationsstufe bei einer Reaktionstemperatur von maximal 50°C einverleibt und die Kondensation in mehreren Stufen zu Ende führt,

d) das erhaltene Kondensationsgemisch mit 0,5 bis 1,0 Teilen Anilin pro Teil Kondensationsgemisch versetzt und diese Mischung einer Extraktion mit Wasser unterwirft, wobei 0,8 bis 1,5 Teile Wasser auf ein Teil Anilin/Kondensationsgemisch-Mischung verwendet werden,

e) die wässrige Extraktionsphase auf einen Aminhydrochloridgehalt von mindestens 80% der Sättigungsgrenze bei 25°C einengt und an den Prozessanfang zurückführt und

f) aus der organischen Phase die Polyphenyl-polymethylen-polyamine isoliert.

2. Verfahren nach Anspruch 1, dadurch gekennzeichnet, dass man die mehrstufige Kondensation in 4 Stufen durchführt, wobei die Reaktionstemperatur in der ersten Stufe 20° bis 50°C, in der zweiten Stufe 50° bis 60°C, in der dritten Stufe 70° bis 80°C und in der vierten Stufe 90° bis 110°C ist und die Verweilzeit in jeder Stufe 0,5 bis 1,0 Stunden beträgt.

3. Verfahren nach Anspruch 1, dadurch gekennzeichnet, dass man als Katalysator eine bei 25°C ge-sättigte, wässrige Aminhydrochloridlösung verwen-det.

4. Verfahren nach Anspruch 3, dadurch gekennzeichnet, dass die Aminhydrochloride im wesentlichen bestehen aus Polyphenyl-polymethylen-polyamin-hydrochloriden und Anilinhydrochlorid.

5. Verfahren nach Anspruch 4, dadurch gekennzeichnet, dass das Aminhydrochlorid, bezogen auf das Gesamtgewicht 85 bis 95 Gew.-% Anilinhydrochlorid enthält.

## Claims

1. A process for the preparation of polyphenyl polymethylene polyamines containing a large amount of diamino-diphenylmethane isomers and a large amount of 4,4'-diamino-diphenylmethane by multistage condensation of aniline and formaldehyde in the presence of an amine hydrochloride as catalyst, wherein

a) the starting materials aniline, formaldehyde and hydrogen chloride are used in such quantities that the molar ratio of aniline to formaldehyde is from 2.5 : 1 to 3 : 1 and the molar ratio of hydrogen chloride to aniline is from 0.4 : 1 to 0.9 : 1,

b) an aqueous amine hydrochloride solution which is at least 80 percent saturated at 25°C and which contains an amount of aniline hydrochloride of between 85 and 100 percent by weight, based on the amine hydrochloride content, is used as catalyst,

c) aniline, formaldehyde and the aqueous amine hydrochloride solution are continuously incorporated into the reaction mixture of the first condensation stage at a reaction temperature of not more than 50°C, and the condensation is completed in a plurality of stages,

d) the resulting condensatin mixture is mixed with 0.5 part to 1.0 part of aniline per part of condensation mixture and this mixture is extracted with water, from 0.8 part to 1.5 parts of water being employed per part of the mixture of aniline and condensation mixture,

e) the aqueous extraction phase is concentrated to an amine hydrochloride content of at least 80 percent of the saturation limit at 25°C, and the concentrated aqueous extraction phase is recycled to step (a), and

f) the polyphenyl polymethylene polyamines are isolated from the organic phase.

2. A process as claimed in claim 1, wherein the multistage condensation is carried out in four stages, the reaction temperature in the first stage being between 20°C and 50°C, in the second stage between 50°C and 60°C, in the third stage between 70°C and 80°C, and in the fourth stage between 90°C and 110°C, and the residence time in each stage being between 0.5 hour and 1.0 hour.

3. A process as claimed in claim 1, wherein the catalyst used is an aqueous amine hydrochloride solution which is saturated at 25°C.

4. A process as claimed in claim 3, wherein the amine hydrochlorides consist essentially of poly-phenylpolymethylene polyamine hydrochlorides and aniline hydrochloride.

5. A process as claimed in claim 4, wherein the amine hydrochloride contains from 85 to 95 percent by weight, based on the total weight of the amine hydrochloride, of aniline hydrochloride.

## Revendications

1. Procédé de préparation de polyphényl-polymé-thylène-polyamines à teneur importante en isomères de diamino-diphénylméthane et à proportion élevée en 4,4-diamino-diphénylméthane, par condensation en plusieurs étapes d'aniline et de formaldéhyde, en présence de chlorhydrate d'amine comme catalyseur, caractérisé par le fait que

a) on utilise les matières composantes, aniline, formaldéhyde et acide chlorhydrique, en quantités telles que le rapport molaire, aniline/formaldéhyde, sont de 2,5 à 3/1 et celui, acide chlorhydrique/anili-ne, de 0,4 à 0,9/1,

b) on utilise, comme catalyseur, une solution aqueuse de chlorhydrate d'amine saturée, à 25°C, à au moins 80%, et qui contient, par rapport à la teneur en chlorhydrate d'amine, 85 à 100% en poids de chlorhydrate d'aniline,

c) on incorpore l'aniline, le formaldéhyde et la solution aqueuse de chlorhydrate d'amine, en continu, au mélange de réaction de la première étape de condensation, à une température de réaction de 50°C au maximum et on mène la condensatin à sa fin en plusieurs étapes,

d) on additionne le mélange de condensation avec 0,5 à 1,0 partie d'aniline par partie de mélange de condensation et on soumet ce mélange à une extraction à l'eau, en utilisant 0,8 à 1,5 partie d'eau sur une partie de mélange aniline/mélange de condensation,

e) on concentre la phase d'extraction aqueuse à une teneur en chlorhydrate d'amine d'au moins 80% de la limite de saturation, à 25°C, et on recycle à l'entrée du processus, et

f) on isole de la phase organique les polyphényl--polyméthylène-polyamines.

2. Procédé selon la revendication 1, caractérisé par le fait que l'on effectue la condensation en 4 étapes, la température de réaction étant de 20° à 50°C dans la première étape, de 50° à 60°C dans la deuxième étape, de 70° à 80°C dans la troisième étape, et de 90° à 110°C dans la quatrième étape, et la durée de séjour dans chaque étape étant de 0,5 à 1,0 heure.

3. Procédé selon la revendication 1, caractérisé par le fait que l'on utilise, comme catalyseur, une solution aqueuse de chlorhydrate d'amine saturée à 25°C.

4. Procédé selon la revendication 3, caractérisé par le fait que les chlorhydrates d'amine sont constitués éventuellement de chlorhydrates de polyphényle-polyméthylène-polyamines et de chlorhydrates d'aniline.

5. Procédé selon la revendication 4, caractérisé par le fait que le chlorhydrate d'amine contient, rapporté au poids total, 85 à 95% en poids de chlorhydrate d'aniline.